# EUROPEAN PATENT SPECIFICATION

(11) **EP 2 416 789 B1**
(45) Date of publication and mention of the grant of the patent: **21.06.2017**
(21) Application number: 10713442.1
(22) Date of filing: 07.04.2010
(51) Int. Cl.: A61K 35/12, A61M 1/00

(54) **METHOD AND DEVICE FOR TREATMENT OF CONDITIONS ASSOCIATED WITH INFLAMMATION OR UNDESIRABLE ACTIVATION OF THE IMMUNE SYSTEM**
VERFAHREN UND VORRICHTUNG ZUR BEHANDLUNG VON ERKRANKUNGEN IM ZUSAMMENHANG MIT ENTZÜNDUNG ODER UNERWÜNSCHTER AKTIVIERUNG DES IMMUNSYSTEMS
MÉTHODE ET DISPOSITIF DE TRAITEMENT DE PATHOLOGIES ASSOCIÉES À L'INFLAMMATION OU À L'ACTIVATION INDÉSIRABLE DU SYSTÈME IMMUNITAIRE

(30) Priority: 07.04.2009 DK 200900471; 07.04.2009 US 167333 P; 10.09.2009 EP 09169937
(43) Date of publication of application: 15.02.2012
(73) Proprietor: Velin-Pharma A/S, 3480 Fredensborg (DK)
(72) Inventor: LINDENBERG, Svend, 2942 Skodsborg (DK); VELIN, Flemming, 3480 Fredensborg (DK)
(74) Representative: Inspicos P/S
(86) International application number: PCT/EP2010/054590
(87) International publication number: WO 2010/118979

(56) References cited:
- EP-A2- 0 257 962
- WO-A1-00/46249
- WO-A1-92/07083
- WO-A1-99/09051
- WO-A1-2007/090569
- MEIJER H ET AL: "The production of anti-inflammatory cytokines in whole blood by physico-chemical induction" IMFLAMMATION RESEARCH, BIRKHAEUSER VERLAG, BASEL LNKD- DOI:10.1007/S00011-003-1197-1, vol. 52, no. 10, 1 October 2003 (2003-10-01), pages 404-407, XP002434400 ISSN: 1023-3830

## Description

### FIELD OF THE INVENTION

The present invention relates to methods and compositions for use in the treatment of specific medical conditions. The compositions of the invention comprise blood serum preparations, such as activated blood serum preparations. The present invention also relates to the use of such blood serum preparations for the treatment of diseases and disorders associated with colitis ulcerosa.

### BACKGROUND OF THE INVENTION

Inflammation, a key component of the immune system, functions in both defense (physiological) and in pathophysiological events to maintain the homeostasis of tissues, organs and individual cells. Acute inflammation is a short-term process characterized by the classic signs of inflammation, i.e. swelling, redness, pain, heat, and loss of function, due to infiltration of tissues by plasma of several activated components such as interleukines, antibodies, hormones etc. and leukocytes. It occurs as long as the injurious stimulus is present and ceases once the stimulus has been removed. Chronic inflammation is a pathological condition characterized by concurrent active inflammation, tissue destruction, and attempts at repair. Chronically inflamed tissue is characterized by the infiltration of mononuclear immune cells (monocytes, macrophages, lymphocytes, dendritic cells and other plasma cells), tissue destruction, and attempts at healing, which include angiogenesis and fibrosis.

Without inflammation, wounds and infections would not be able to heal and progressive destruction of the tissue would threaten the survival of the organism. Inappropriate inflammation, on the other hand, can lead to diseases, such as hay fever, atherosclerosis, neurodegenerative diseases such as Alzheimer's, cancer and rheumatoid arthritis. For these reasons, inflammation is tightly regulated by the body.

Inflammation is controlled by more than 400 genes. The pro-inflammatory genotype, which appears dominant, increases our vulnerability to, and intensity of, inflammatory reactions, which underlie chronic inflammatory diseases, especially in old age. (Ferencik et al., Inflammation--a lifelong companion. Folia Microbiol (Praha). 2007; 52:159-73).

Mononuclear immune cells are under infectious conditions attracted to the site of infection in an attempt to eliminate the foreign pathogen through phagocytosis. Leukocytes and dendritic cells are here activated by the pathogens to synthesize and release proinflammatory cytokines such as IL- 1 β, IL- 3, IL-5, IL-6, IL-8, TNF-α (tumor necrosis factor-α), GM-CSF (granulocyte-macrophage colony-stimulating factor), and MCP- I (monocyte chemotactic protein- 1). These released cytokines then further attract more immune cells to the infected site, amplifying the response of the immune system to defend the host against the foreign pathogen.

Recently, biological agents that modulate the pro-inflammatory activities of TNF-α and IL- lβ have shown efficacy as novel anti-inflammatory drugs (Evans and Robbins, J. Rheumatol. 21:779-782 (1994); Robbins and Evans, Gene Ther. 3:187-189 (1996); Evans and Robbins, Curr Opin Rheumatol. 8:230-234 (1996); Evans et al., Arthritis Rheum. 42:1-16 (1999); Ghivizzani et al., Clin Orthop. 379 (Suppl):S288-299 (2000)).

Dendritic cells are derived from hemopoietic bone marrow progenitor cells. These progenitor cells initially transform into immature dendritic cells. These cells are characterized by high endocytic activity and low T-cell activation potential. Immature dendritic cells constantly sample the surrounding environment for foreign pathogens. This is done through pattern recognition receptors such as the toll-like receptors (TLRs), which recognize specific chemical signatures found on subsets of pathogens. Once they have come into contact with a presentable antigen, they become activated into mature dendritic cells and begin to migrate to the lymph node. Immature dendritic cells phagocytose pathogens and degrade their proteins into small pieces and upon maturation present those fragments at their cell surface using MHC molecules. Simultaneously, they upregulate cell-surface receptors that act as co-receptors in T-cell activation. Once in the lymph nodes they act as antigen-presenting cells, in activating helper T-cells and killer T-cells as well as B-cells by presenting them with antigens derived from the pathogen, together with non-antigen specific co-stimulatory signals.

WO 06007529 relates to the use of exosome preparations isolated directly from serum for the treatment of diseases and disorders associated with undesirable activation of the immune system.

WO 07090569 relates to methods for producing conditioned blood compositions and to the use for the treatment of an illness of the human or animal body.

WO 03080122 relates to a method for producing induced blood compositions from blood, whereby the blood cells, in a transient or stable manner, express and optionally secrete one or more therapeutically and/or diagnostically significant proteins and/or effector molecules.

WO 0046249 relates to a method for producing interleukin 1 receptor antagonists, wherein a syringe is being filled with a body fluid and incubated, and a prophylactically or therapeutically active protein subsequently is being formed in the body fluid.

WO 9909051 relates to a syringe for inducing therapeutically-effective proteins.

Glucocorticoids (also referred to as "corticosteroids" or "steroidal drugs") represent today one of the most effective clinical treatment for a range of inflammatory conditions, including acute inflammation. However, steroidal drugs can have side effects that may threaten the overall health of the patient.

Thus, there remains a need to develop a safe, effective method of treating autoimmune diseases and/or inflammatory disorders. The present invention provides compositions and methods for treating such diseases and disorders.

### OBJECT OF THE INVENTION

It is an object of embodiments of the invention to provide compositions and methods for the treatment of medical conditions, such as medical conditions associated with chronic inflammation and/or as medical conditions such as colitis ulcerosa.

### SUMMARY OF THE INVENTION

It has been found by the present inventor(s) that a blood serum preparation may be used in the treatment of new medical conditions, such as medical conditions associated with tissues derived from the endoderm, or so-called endoderm-related medical condition. In some aspects of the invention the medical condition or disorder is a medical condition or disorder in which an important pathogenetic role is assigned to inflammation. In some other aspects of the invention the medical condition or disorder is associated with autoimmunogenic activity.

It has further been found by the present inventors that specific miRNA are upregulated in body fluids or element thereof upon activation or stimulation of cells or cell components in this body fluid or its elements and that compositions comprising miRNAs may be used for the treatment or for alleviating the symptoms of a disease, disorder or dysfunctions in the body, such as conditions associated with inflammation, a disease of the immune system, such as undesirable activation of the immune system and/or cancer or other indications associated with abnormal cell growth or cell division.

So, in a first aspect the present invention relates toof an activated blood serum preparation or an activated a whole blood preparation for use in the treatment or for alleviating the symptoms of colitis ulcerosa, in a mammal.

In a second aspect the present invention relates to a composition comprising an activated, blood serum preparation or an activated a whole blood preparation for use in the treatment or for alleviating the symptoms of colitis ulcerosa in a mammal.

In further aspects of the invention the method comprises a step, wherein the blood serum preparation is further purified by the use of immune beads. Such immune beads may be coated with antibodies specific for a particular plasma or cell surface proteins. Alternatively or in addition to, the immune beads may be coated with cell adhesion molecules, polysaccharides to stimulate production of substances from the cells in the body fluid, or substances lysing specific cells to expel intracellular components.

In further aspects of the invention the method comprises a step, wherein the buffer coat is separated from the blood serum preparation. As used herein "buffer coat" refers to the layer obtained after centrifugation of blood comprising thrombocytes, leucocytes and other non-haemoglobin containing cells

In further aspects of the invention the collected blood is incubated in contact with an increased surface area for a specific amount of time within 2 to 48, such 2 to 26 hours, such as for at least about 5 hours, such as for at least about 6 hours, such as for less than about 24 hours, such as for less than about 20 hours. The specific optimal time of incubation may vary dependent on specific diseases to be treated, amount of blood collected, the type and extend of blood stimulation, and may be optimized accordingly.

In further aspects of the invention, the collected blood is incubated in contact with an increased surface area by the presence of beads, scaffolding, or similar structures of glass, plastic material or other suitable material, which increases the surfaces exposed to the blood components.

In further aspects of the invention, the mammalian blood serum is activated prior to collection from this mammal.

Accordingly in some embodiments of the invention monocyte activating substances, such as an inductor or enhancing agent as defined herein may be given to the patient prior to collection of the blood.

In particular embodiments of the invention blood is not collected from persons or other mammals already having a fever or virus or bacterial infection.

In further aspects of the invention, during the incubation of the collected blood in contact with an increased surface area, further surface expanding substances are added such as beads, scaffolding, or similar structures of glass, plastic material or other suitable material, which further increases the surfaces exposed to the blood components.

In further aspects of the invention, an anticoagulant is added to the blood collected from the mammal before incubation in contact with an increased surface area. This may improve the harvest of the active substances as monocytes are not trapped within the clotted blood material. Further the coagulation process might jeopardize the induction for preparation of the active substances in the blood by activation of the complement system potentially destroying small membrane bound items such as proteins, receptors or RNA.

In a further aspect, the invention provides a device for preparing an activated body fluid composition, the device comprising a vessel with an inductor, wherein the vessel has a wall structure formed continuously about an internal space and an entry point provided in a top end of the wall for injecting the body fluid composition into the internal space.

The vessel could e.g. be made of glass or plastic, e.g. polycarbonate or PVC etc.

By continuously is herein meant that the wall is tight against diffusion of the body fluid composition at least for a period exceeding a week, and preferably for a period exceeding a year. Accordingly, the wall has no openings, and the body fluid composition must be injected into the inner space via entry point. For that purpose, the entry point may comprise an element of an elastically deformable material which can be pierced by a cannula for therapeutic injection.

In a further aspect the present invention relates to a composition comprising a therapeutically effective amount of one or more nucleic acid molecule encoding a miRNA or functional variant thereof, said miRNA being upregulated in a body fluid or element thereof upon activation of said body fluid or element thereof; for the preparation of a medicament.

### DETAILED DISCLOSURE OF THE INVENTION

### Definitions

The term "endoderm-related medical condition" as used herein refers to a medical condition in a tissue or involving cells or tissues derived from the endoderm.

The terms "paradentosis" or "periodontitis" as used herein, refers to an inflammatory disease that affects the periodontium within the oral cavity. In some embodiments the paradentosis is associated with localized pain, erythema, swelling, loosening of teeth, and dental pockets.

The term "abortus habitualis" also known as "miscarriage" as used herein refers to the medical condition of repeated spontaneous termination of a pregnancy by the expulsion of an embryo or fetus from the uterus before the 20th week of gestation (often for no known reason). This condition is in the present embodiment also refered to unexplained infertility were the blastocysts fail to attach and implant in the endometrium due to an imbalance in factors that is corrected by this invention.

The terms "colitis ulcerosa" or "ulcerative colitis" as used herein refers to a chronic inflammatory disease of the large intestine and/or rectum. The colitis ulcerosa is often characterized by recurrent episodes of abdominal pain and fever and chills and profuse diarrhea.

The term "polymyalgia rheumatica" as used herein refers to the clinical syndrome characterized by severe aching and stiffness in the neck, shoulder girdle, and pelvic girdle, usually causing severe pain in the proximal muscle groups.

The terms "whiplash" or "whiplash-associated disorders" as used herein refers to a range of injuries to the neck caused by or related to a sudden distortion of the neck. In some embodiments the whiplash is associated with motor vehicle accidents, falls from bicycles or horses or head banging.

The term "endometriosis" as used herein refers to the general condition in women in which endometrial cells are deposited in areas outside the uterine cavity. Endometrial cells deposited in areas outside the uterus (endometriosis) may give symptoms of pelvic pain and may give rice to infertility.

The terms "adenomyosis" or "adeomyosis" as used herein refers to the condition in women in which endometrial cells are positioned within the myometrium of the uterus outside the endometrial cavity. This may cause bleeding, pain and infertility.

The term "autologous" as used herein refers to blood serum preparation that are administered to the same individual as they come from. In a preferred embodiment, the blood serum preparation is autologous to the mammal in need of said treatment.

However, the blood serum preparation may also be derived from a genetically non-identical member of the same species, such as another human being. In this case the blood serum preparation is referred to as allogeneic or homologous.

In some embodiments the composition suitable for therapeutic application derives from another species, i.e. a heterologous preparation or a xenograft or xenogenic preparation. This may be a heterologous preparation from similar or closely related mammals. Accordingly the composition suitable for therapeutic application may be derived from a domestic animal, such as a horse and used in another mammal, such as in a human being.

In some embodiments the blood serum preparation derives from another species, i.e. a xenograft preparation. This may be xenograft preparation from similar or closely related mammals.

The term "body fluid composition" as used herein refers to any fluid that may be obtained from the body of a mammal. Included within this definition are cerebrospinal fluids, blood, such as blood from the circulatory system or from the umbilical cord, serum, lymph fluid, plasma, pleura exudates, peritoneal exudates, bone marrow exudates, extracellular fluids, fluids from the joints, amniotic fluids. Included within this definition are also cells, such hematopoietic stem cells or in vitro cell cultures, such as a monocyte cell cultures, as well as exosomes or other substructures that may be derived from a body fluid and a conditioned cell culture medium.

The term "conditioned cell culture medium" as used herein refers to a medium, such as a growth medium wherein cells have been cultured for a period of time, such as by in vitro cultivation. The period of time for culturing may be 1, 2, 4, 8, 16, 24, 48, 72 hours or as long as the cells are viable and stabile.

In one embodiment, the body fluid according to the invention comprises leukocytes, such as monocytes and dendritic cells.

Bone marrow exudates may be obtained by bone marrow aspiration, wherein an amount of bone marrow (such as from the hip) is removed through a needle. The needle is placed through the top layer of bone and a liquid sample containing bone marrow cells is obtained by aspirating it into a syringe. The bone marrow exudates may further be centrifuged to obtain a fraction containing blood cells.

In one preferred embodiment, the body fluid is a serum, derived from blood of the circulatory system.

The term "blood serum preparation" as used herein refers to a preparation comprising the liquid part of blood derived from a mammal. In some embodiments blood serum preparation does not contain significant levels of intact blood cells, such as monocytes or red blood cells. In some embodiments, blood serum preparation is lacking significant levels of clotting factors. The "blood serum preparation" may in some embodiments contain clotting factors and may in this case be referred to as a blood plasma preparation.

In some embodiments, the blood serum preparation is activated.

The term "activated" or "conditioned" as used herein refers to the treatment of whole blood in vitro or in vivo, or other suitable body fluid for a period of time in a container outside the living body, such as in a container comprising a surface that is able to trigger an immunological response in the monocytes, such as leucocytes or dendritic cells of blood preparation. In some embodiment the whole blood is activated by exposure to an enhancing agent, or by stimulation to express an enhancing agent.

In some embodiment the blood serum preparation is prepared by a method as disclosed in any one of international patent applications WO06007529, WO07090569, WO03080122, WO0046249, or WO9909051.

The term "inductor" or "enhancing agent" as used herein refers to any structure, substance or compound that may be used to induce maturation or activation of the body fluid composition, such as a blood serum preparation used according to the invention, such in activation in antigen presenting cells (APCs). In some embodiments the inductor is a biological compound such as immunoglobulins that are able to induce or potentiate an immunological response in leukocytes or dendritic cells of the blood preparation.

The term "anticoagulant" as used herein refers to any substance that prevents coagulation. Included within this definition is Warfarin (Coumadin), Acenocoumarol, phenprocoumon, Phenindione, Heparin, Low molecular weight heparin, Synthetic inhibitors of factor Xa, such as Fondaparinux and Idraparinux, thrombin inhibitors, such as argatroban, lepirudin, bivalirudin, and dabigatran.

The term "Platelet-rich plasma" or "PRP", as used herein refers to a concentrated source of platelets, such as autologous platelets. PRP is known to contain and also releases (through degranulation) several growth factors (cytokines) that stimulate soft tissue healing.

In some embodiments, the body fluid used according to the invention is an activated blood serum preparation that has been mixed with platelet-rich plasma (PRP). This may be used for several types of medical disorders or conditions, such as wound healing, such as associated with surgery, tendonitis, cardiac care, cartilage regeneration, disc regeneration, and dental health.

As used herein, the term "cancer" includes, but is not limited to, solid tumors and blood borne tumors, including leukemia and lymphoma. The term cancer refers to diseases of the skin, tissues, organs, bone, cartilage, blood and vessels. The term "cancer" further encompasses primary and metastatic cancers.

As used herein, the term "miRNA" or "miR" or "microRNA" means a non-coding RNA between 17 and 25 nucleobases in length which hybridizes to and regulates the expression of a coding RNA. A 17-25 nucleotide miRNA molecule can be obtained from a miR precursor through natural processing routes (e.g., using intact cells or cell lysates) or by synthetic processing routes (e.g., using isolated processing enzymes, such as isolated Dicer, Argonaut, or RNAase III). It is understood that the 17- 25 nucleotide RNA molecule can also be produced directly by biological or chemical syntheses, without having been processed from a miR precursor.

As used herein, the term "miR precursor," "pre-miRNA" or "pre-miR" means a non-coding RNA having a hairpin structure, which contains a miRNA. In certain embodiments, a pre-miRNA is the product of cleavage of a primary mi-RNA transcript, or "pri-miR" by the double-stranded RNA-specific ribonuclease known as Drosha, but a pre-miRNAs can also be produced directly by biological or chemical synthesis without having been processed from a pri-miR.

### MicroRNA

The present invention is directed to compositions and methods related to the use of nucleic acid molecule encoding miRNAs in the treatment of an indication selected from the list consisting of a disease or disorder associated with inflammation, a disease of the immune system, such as undesirable activation of the immune system, cancer or other indications associated with abnormal cell growth or cell division.

It is well known in the art that modifications can be made to the sequence of a miRNA or a pre-miRNA or pri-miRNA without disrupting miRNA activity. As used herein, the term "functional variant" of a miRNA sequence refers to an oliginonucleotide sequence that varies from the natural miRNA sequence, but retains one or more functional characteristics of the miRNA (e.g. cancer cell proliferation inhibition, induction of cancer cell apoptosis, enhancement of cancer cell susceptibility to chemotherapeutic agents, specific miRNA target inhibition). In some embodiments the "functional variants" refers to a miRNA that vary by one or two nucleotides, such as one or two substitutions, additions, deletions or combinations thereof. In some embodiments, a functional variant of a miRNA sequence retains all of the functional characteristics of the miRNA. In certain embodiments, a functional variant of a miRNA has a nucleobase sequence that is a least about 60%, 65%, 70%, 75%, 80%, 85%, 90%, 91%, 92%, 93%, 94%, 95%, 96%, 97%, 98% or 99% identical to the miRNA or precursor thereof over a region of about 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases, or that the functional variant hybridizes to the complement of the miRNA or precursor thereof under stringent hybridization conditions. Accordingly, in certain embodiments the nucleobase sequence of a functional variant may is capable of hybridizing to one or more target sequences of the miRNA.

It is understood that any nucleobase sequence set forth herein are independent of any modification to a sugar moiety, an internucleoside linkage, or a nucleobase. It is further understood that a nucleobase sequence comprising U's also encompasses the same nucleobase sequence wherein "U" is replaced by "T at one or more positions having "U." Conversely, it is understood that a nucleobase sequence comprising T's also encompasses the same nucleobase sequence wherein "T; is replaced by "U at one or more positions having "T".

Nucleobase sequences miRNAs and their corresponding stem-loop sequences described herein may be found in miRBase, an online searchable database of miRNA sequences and annotation, found at http://microrna.sanger.ac.uk/. Entries in the miRBase Sequence database represent a predicted hairpin portion of a miRNA transcript (the stem-loop), with information on the location and sequence of the mature miRNA sequence. The miRNA stem-loop sequences in the database are not strictly precursor miRNAs (pre-miRNAs), and may in some instances include the pre-miRNA and some flanking sequence from the presumed primary transcript. The miRNA nucleobase sequences described herein encompass any version of the miRNA, including the sequences described in Release 10.0 of the miRBase sequence database and sequences described in any earlier Release of the miRBase sequence database. A sequence database release may result in the re-naming of certain miRNAs. A sequence database release may result in a variation of a mature miRNA sequence.

The present invention pertains to pharmaceutical compositions containing nucleic acid molecules. As used herein, the term "nucleic acid molecule" is intended to include DNA molecules and RNA molecules and analogs of the DNA or RNA generated using nucleotide analogs. The nucleic acid molecule can be single-stranded or double-stranded, such as in the form of Small interfering RNA (siRNA) or double stranded miRNA (dsmiRNA), and may be generated using purified enzymes or by chemical synthesis. They may be crude or purified. The term "miRNA," unless otherwise indicated, refers to the mature miRNA sequence.

In specific embodiments, a nucleic acid molecule of the present invention comprises a nucleotide sequence which is at least about 60%, 65%, 70%, 75%, 80%, 85%, 90%, 91%, 92%, 93%, 94%, 95%, 96%, 97%, 98%, 99% or more identical to the entire length of the miRNA, or a portion of any of these nucleotide sequences.

In specific embodiments, a nucleic acid molecule of the present invention comprises a nucleotide sequence which is less than about 100 nucleotides in length, such as less than about 80 nucleotides in length, such as less than about 60 nucleotides in length, such as less than about 40 nucleotides in length, such as less than about 30 nucleotides in length, such as less than about 25 nucleotides in length, such as less than about 23 nucleotides in length, such as less than about 21 nucleotides in length, such as less than about 19 nucleotides in length.

In specific embodiments, a nucleic acid molecule of the present invention comprises a nucleotide sequence, which is at least about 19 nucleotides, such as at least about 21 nucleotides, such as at least about 23 nucleotides, such as at least about 25 nucleotides, such as at least about 30 nucleotides, such as at least about 40 nucleotides, such as at least about 50 nucleotides, such as at least about 50 nucleotides, such as at least about 60 nucleotides, such as at least about 80 nucleotides, such as at least about 100 nucleotides in length.

A nucleic acid sequence can be RNA or DNA, and can be single or double stranded, and can be selected from a group comprising: RNA nucleotides, DNA nucleotides, and nucleic acid analogues; for example peptide-nucleic acid (PNA), pseudo-complementary PNA (pc-PNA), locked nucleic acid (LNA), etc.

### Specific embodiments of the invention

In some non-limiting embodiments of the invention, blood serum preparation is activated to enhance the anti-inflammatory and/or immunosuppressive activity of the preparation.

Enhancing agents may be cytokines, cytokine antagonists, and NFkappaB antagonists, and include, but are not limited to, TGF-.beta., IL-10, CTLA4-Ig, sCD40-Ig, IL-4, IL-13, FasL, IL-1 receptor antagonist protein (IL-1Ra), vIL-10, sICAM-1, sICAM-3, and TRAIL. In some non-limiting embodiments, the enhancing agent is IL-1Ra, IL-10 or IL-4 or a combination thereof. Optionally, where a specific antigen or a specific antigen source is known, such specific antigen or specific antigen source (e.g., fixed or attenuated infectious agent) may be added to the culture as an enhancing agent in a non-toxic, non-pathogenic amount.

In some embodiments of the invention, peripheral blood is conditioned by incubation in the presence of beads to stimulate the production of cytokines prior to serum collection. Beads which may be used for this purpose include, but are not limited to, glass or plastic beads between 0.5 and 10 mm or between 0.5 and 5 mm in diameter, optionally treated with an agent, such as CrSO₄, which stimulates lymphocyte proliferation (Mignini et al., 2004, Preventive Med 39(4) 767-775; Rhee et al., 2002, Clin Exp Immunol 127(3):463-469). In one preferred, non-limiting embodiment of the invention, glass beads, 2.5 mm in diameter, having a surface area of 21 mm² of medical grade, surface modified by incubation in 50% CrSO₄ (Merck, Germany) for 5 minutes, then washed with distilled water until the pH was the same as that of the distilled water and the conductivity of the wash solution was less then 0.3 µS, may be used. The treated beads may be placed in a suitable container, such as a microtiter plate, centrifuge tube, culture tube, or syringe, and then sterilized (e.g. by autoclaving or gamma irradiation). Peripheral blood may then be introduced into the bead-containing container, and then incubated, aseptically, at 37 °C., 5% CO₂ for example for 24 hours. Serum may then be collected from the bead/blood suspension by centrifugation, for example at 3500 rpm for 10 minutes. Typically, 20 percent of the total original peripheral blood volume may be recovered. The resulting serum containing exosomes may then be stored at -20 °C. Orthokine™ serum is prepared in this way (see U.S. Pat. Nos. 6,759,188 and 6,713,246).

In a related, specific, non-limiting embodiment of the invention, Enhancing agents may be added to the peripheral blood sample prior to, or as an alternative to, incubation with beads. For example, 5 µg enhancing agent per ml of peripheral blood may be added.

In some, non-limiting embodiment of the invention, a blood serum preparation is prepared by collecting serum from peripheral blood, optionally incubated with beads and/or an enhancing agent, by centrifugation to remove the formed blood elements (e.g., at 3000-5000 g for 10 minutes), followed by ultracentrifugation, for example, at 100,000 g, for 1 hours. The resulting pellet may be resuspended in physiologic saline, and then preferably sterilized (e.g., by filtration through a 0.2 µm filter).

The invention provides in a further embodiment for the incubation of the body fluid in the syringe to be carried out over a period of from 12 to 72 hours, preferably 24 hours, preferably at room temperature, that is to say 20 °C. to 41 °C., in particular at 37 °C.

The invention also provides in one configuration of the invention for the body fluid to be treated further after formation of the therapeutically or prophylactically active protein or compound in the body fluid, in order, for example, to remove particular constituents of the latter, for example blood plasma or blood platelets. This removal may in some embodiments of the invention be carried out by centrifugation, filtration or coagulation to remove coagulation factors and/or clotted material. In alternative embodiments of the invention the body fluid composition may be mixed with other body fluids or body fluid components. Accordingly, in some particular embodiments, an activated body fluid composition, such an activated blood serum preparation is mixed with Platelet-rich plasma prior to administration to the patient. This is particularly suitable for use in the treatment of indications associated with joints, tendons, ligaments, and muscles, such as in the treatment of muscle pain, polymyalgia rheumatica and whiplash-associated disorders.

As discussed above the present invention relates to methods for the treatment or for alleviating the symptoms of a medical condition, compositions comprising an activated body fluid composition, such as a blood serum preparation for the preparation of a medicament for the treatment or for alleviating these symptoms, and the use of these compositions in the preparation of medicaments.

In some aspects the present invention relates to a method for the treatment or for alleviating the symptoms of a disease or disorder associated with inflammation, a disease of the immune system, such as undesirable activation of the immune system and/or cancer or other indications associated with abnormal cell growth or cell division, the method comprising administering a composition comprising a therapeutically effective amount of one or more nucleic acid molecule encoding a miRNA or functional variant thereof, said miRNA being upregulated in a body fluid or element thereof upon activation of said body fluid or element thereof to a subject in need of said treatment.

In some embodiments the miRNA is selected from the list consisting of hsa-let-7a, hsa-let-7a-1, hsa-let-7a-2, hsa-let-7a-3, hsa-let-7b, hsa-let-7b*, hsa-let-7c, hsa-let-7d, hsa-let-7d*, hsa-let-7e, hsa-let-7e*, hsa-let-7f, hsa-let-7f-1, hsa-let-7f-2, hsa-let-7g, hsa-let-7i, hsa-miR-1, hsa-miR-1-2, hsa-miR-100, hsa-miR-100-1, hsa-miR-100-2, hsa-miR-101, hsa-miR-101-1, hsa-miR-101a, hsa-miR-101b-2, hsa-miR-102, hsa-miR-103, hsa-miR-103-1, hsa-miR-103-2, hsa-miR-104, hsa-miR-105, hsa-miR-106a, hsa-miR-106a-1, hsa-miR-106b, hsa-miR-106b-1, hsa-miR-107, hsa-miR-10a, hsa-miR-10b, hsa-miR-122a, hsa-miR-1228*, hsa-miR-123, hsa-miR-124a, hsa-miR-124a-1, hsa-miR-124a-2, hsa-miR-124a-3, hsa-miR-125a, hsa-miR-125b, hsa-miR-125b-1, hsa-miR-125b-2, hsa-miR-126, hsa-miR-126-5p, hsa-miR-126*, hsa-miR-127, hsa-miR-128a, hsa-miR-128b, hsa-miR-129, hsa-miR-129-1, hsa-miR-129-2, hsa-miR-130, hsa-miR-130a, hsa-miR-130a-1, hsa-miR-130b, hsa-miR-130b-1, hsa-miR-132, hsa-miR-133a, hsa-miR-133b, hsa-miR-134, hsa-miR-135a, hsa-miR-135b, hsa-miR-136, hsa-miR-137, hsa-miR-138, hsa-miR-138-1, hsa-miR-138-2, hsa-miR-139, hsa-miR-139-5p, hsa-miR-140, hsa-miR-140*, hsa-miR-141, hsa-miR-142-3p, hsa-miR-142-5p, hsa-miR-143, hsa-miR-144, hsa-miR-144*, hsa-miR-145, hsa-miR-146a, hsa-miR-146a*, hsa-miR-146b, hsa-miR-147, hsa-miR-148a, hsa-miR-148b, hsa-miR-149, hsa-miR-15, hsa-miR-150, hsa-miR-151, hsa-miR-151*, hsa-miR-152, hsa-miR-153, hsa-miR-154, hsa-miR-154*, hsa-miR-155, hsa-miR-15a, hsa-miR-15a-2, hsa-miR-15b, hsa-miR-16, hsa-miR-16-1, hsa-miR-16-2, hsa-miR-16a, hsa-miR-164, hsa-miR-170, hsa-miR-172a-2, hsa-miR-17, hsa-miR-17-3p, hsa-miR-17-5p, hsa-miR-17-92, hsa-miR-18, hsa-miR-18a, hsa-miR-18b, hsa-miR-18a*, hsa-miR-181a, hsa-miR-181a-1, hsa-miR-181a-2, hsa-miR-181a*, hsa-miR-181a-1*, hsa-miR-181b, hsa-miR-181b-1, hsa-miR-181b-2, hsa-miR-181c, hsa-miR-181d, hsa-miR-182, hsa-miR-182*, hsa-miR-183, hsa-miR-184, hsa-miR-185, hsa-miR-186, hsa-miR-188, hsa-miR-189, hsa-miR-190, hsa-miR-191, hsa-miR-192, hsa-miR-192-1, hsa-miR-192-2, hsa-miR-192-3, hsa-miR-193a, hsa-miR-193b, hsa-miR-194, hsa-miR-195, hsa-miR-195*, hsa-miR-196a, hsa-miR-196a-2, hsa-miR-196b, hsa-miR-197, hsa-miR-198, hsa-miR-199a, hsa-miR-199a-1, hsa-miR-199a-1-5p, hsa-miR-199a-2, hsa-miR-199a-2-5p, hsa-miR-199a-3p, hsa-miR-199b, hsa-miR-199b-5p, hsa-miR-19a, hsa-miR-19b, hsa-miR-19b-1, hsa-miR-19b-2, hsa-miR-200a, hsa-miR-200b, hsa-miR-200c, hsa-miR-202, hsa-miR-203, hsa-miR-204, hsa-miR-205, hsa-miR-206, hsa-miR-207, hsa-miR-208, hsa-miR-20a, hsa-miR-20b, hsa-miR-21, hsa-miR-210, hsa-miR-211, hsa-miR-212, hsa-miR-213, hsa-miR-214, hsa-miR-215, hsa-miR-216, hsa-miR-217, hsa-miR-218, hsa-miR-218-2, hsa-miR-219, hsa-miR-219-1, hsa-miR-22, hsa-miR-220, hsa-miR-221, hsa-miR-222, hsa-miR-223, hsa-miR-224, hsa-miR-23a, hsa-miR-23b, hsa-miR-24, hsa-miR-24-1, hsa-miR-24-2, hsa-miR-25, hsa-miR-26a, hsa-miR-26a-1, hsa-miR-26a-2, hsa-miR-26b, hsa-miR-27a, hsa-miR-27b, hsa-miR-28, hsa-miR-296, hsa-miR-298, hsa-miR-299-3p, hsa-miR-299-5p, hsa-miR-29a, hsa-miR-29a-2, hsa-miR-29b, hsa-miR-29b-1, hsa-miR-29b-2, hsa-miR-29c, hsa-miR-301, hsa-miR-302, hsa-miR-302a, hsa-miR-302b, hsa-miR-302b*, hsa-miR-302c, hsa-miR-302c*, hsa-miR-302d, hsa-miR-30a, hsa-miR-30a-3p, hsa-miR-30a-5p, hsa-miR-30b, hsa-miR-30b*, hsa-miR-30c, hsa-miR-30c-1, hsa-miR-30d, hsa-miR-30e, hsa-miR-30e*, hsa-miR-30e-5p, hsa-miR-31, hsa-miR-32, hsa-miR-32*, hsa-miR-320, hsa-miR-320-2, hsa-miR-320a, hsa-miR-323, hsa-miR-324-3p, hsa-miR-324-5p, hsa-miR-325, hsa-miR-326, hsa-miR-328, hsa-miR-328-1, hsa-miR-33, hsa-miR-330, hsa-miR-331, hsa-miR-335, hsa-miR-337, hsa-miR-337-3p, hsa-miR-338, hsa-miR-338-5p, hsa-miR-339, hsa-miR-339-5p, hsa-miR-34a*, hsa-miR-340, hsa-miR-340*, hsa-miR-341, hsa-miR-342, hsa-miR-342-3p, hsa-miR-345, hsa-miR-346, hsa-miR-347, hsa-miR-34a, hsa-miR-34b, hsa-miR-34c, hsa-miR-351, hsa-miR-352, hsa-miR-361, hsa-miR-362, hsa-miR-363, hsa-miR-355, hsa-miR-365, hsa-miR-367, hsa-miR-368, hsa-miR-369-5p, hsa-miR-370, hsa-miR-371, hsa-miR-372, hsa-miR-373, hsa-miR-373*, hsa-miR-374, hsa-miR-375, hsa-miR-376a, hsa-miR-376b, hsa-miR-377, hsa-miR-378, hsa-miR-379, hsa-miR-381, hsa-miR-382, hsa-miR-383, hsa-miR-409-3p, hsa-miR-419, hsa-miR-422a, hsa-miR-422b, hsa-miR-423, hsa-miR-424, hsa-miR-429, hsa-miR-431, hsa-miR-432, hsa-miR-432*, hsa-miR-433, hsa-miR-449a, hsa-miR-451, hsa-miR-452, hsa-miR-483, hsa-miR-483-3p, hsa-miR-484, hsa-miR-485-5p, hsa-miR-485-3p, hsa-miR-486, hsa-miR-487b, hsa-miR-451, hsa-miR-452, hsa-miR-452*, hsa-miR-491, hsa-miR-492, hsa-miR-493-3p, hsa-miR-493-5p, hsa-miR-494, hsa-miR-495, hsa-miR-497, hsa-miR-498, hsa-miR-5, hsa-miR-501, hsa-miR-503, hsa-miR-508, hsa-miR-509, hsa-miR-510, hsa-miR-511, hsa-miR-512-5p, hsa-miR-513, hsa-miR-513-1, hsa-miR-513-2, hsa-miR-515-3p, hsa-miR-516-5p, hsa-miR-516-3p, hsa-miR-518a-2*, hsa-miR-518b, hsa-miR-518c*, hsa-miR-519a, hsa-miR-519d, hsa-miR-520c, hsa-miR-521, hsa-miR-524*, hsa-miR-525*, hsa-miR-532-5p, hsa-miR-539, hsa-miR-542-3p, hsa-miR-542-5p, hsa-miR-550, hsa-miR-551a, hsa-miR-561, hsa-miR-563, hsa-miR-565, hsa-miR-572, hsa-miR-582, hsa-miR-584, hsa-miR-594, hsa-miR-595, hsa-miR-598, hsa-miR-600, hsa-miR-601, hsa-miR-602, hsa-miR-605, hsa-miR-608, hsa-miR-611, hsa-miR-612, hsa-miR-615, hsa-miR-615-3p, hsa-miR-622, hsa-miR-627, hsa-miR-628, hsa-miR-635, hsa-miR-637, hsa-miR-638, hsa-miR-642, hsa-miR-648, hsa-miR-652, hsa-miR-654, hsa-miR-657, hsa-miR-658, hsa-miR-659, hsa-miR-662, hsa-miR-663, hsa-miR-7, hsa-miR-7-1, hsa-miR-7-1*, hsa-miR-7-2, hsa-miR-7-3, hsa-miR-708, hsa-miR-765, hsa-miR-769-3p, hsa-miR-802, hsa-miR-885-3p, hsa-miR-9, hsa-miR-9-1, hsa-miR-9-3, hsa-miR-9*, hsa-miR-9-3p, hsa-miR-92, hsa-miR-92-1, hsa-miR-92-2, hsa-miR-9-2, hsa-miR-92, hsa-miR-92a, hsa-miR-93, hsa-miR-95, hsa-miR-96, hsa-miR-98, hsa-miR-99a, and hsa-miR-99b and variant thereof.

In some embodiments the miRNA is selected from the list consisting of hsa-let-7a, hsa-let-7b, hsa-let-7b*, hsa-let-7c, hsa-let-7d, hsa-let-7e, hsa-let-7f, hsa-let-7g, hsa-let-7i, hsa-miR-18a, hsa-miR-205, hsa-miR-126, hsa-miR-34a, hsa-miR-29c, hsa-miR-133a, hsa-miR-15a, hsa-miR-15b, hsa-miR-15b*, and hsa-miR-16.

In some aspects of the invention, body fluid composition, such as peripheral blood is removed from an individual, such as with a syringe, an thereafter transferred to a vessel, wherein the body fluid composition, such as blood is activated, such as by incubation in the presence of beads. Alternatively, the body fluid composition, such as peripheral blood may be removed directly into a vessel, such as a vessel comprising beads to activate the body fluid composition. The activated body fluid composition, such as activated serum may then be collected and used according to the invention.

In still another alternative peripheral blood is removed from an individual, the buffy coat and/or serum is harvested from the blood preparation and transferred to a vessel, such as a vessel comprising beads to activate the body fluid composition.

In some aspects of the invention, the blood serum preparation is activated in syringe or by alternative methods. In some alternative embodiments the blood serum preparation is replaced with another body fluid selected from lymph fluid, saliva or urine. Accordingly, in some alternative embodiments, the syringe is filled with a body fluid selected from lymph fluid, saliva or urine, and treated by methods similar or identical to methods used for treating blood serum. Preferably the body fluid is taken with the syringe directly from the patient.

In some aspects the medical condition or disorder is a medical condition or disorder in which an important pathogenetic role is assigned to inflammation, such as any one disorder listed in table 1.

### Table 1 Examples of inflammatory disorders

Disorders in which an important pathogenetic role is assigned to inflammation:
Alzheimer's disease
Osteoarthritis
Anaphylaxis
Pemphigus
Ankylosing spondylitis
Periodic fever syndromes
Asthma
Psoriasis
Atherosclerosis
Atopic dermatitis
Sarcoidosis
Chronic obstructive pulmonary disease
Crohn's disease (regional enteritis)
Gout
Hashimoto's thyroiditis
Ischaemia-reperfusion injury (occlusive and embolic stroke and myocardial infarction)
Multiple sclerosis
Rheumatoid arthritis
Systemic lupus erythematosus
Type I diabetes mellitus
Ulcerative colitis
Vasculitides (Wegener's syndrome, Goodpasture's syndrome, gaint cell arteritis, polyarteritis nodosa)
Xenograft rejection

Diseases of infectious origin in which inflammation may contribute as much to pathology as does microbial toxicity:
Bacterial dysentery
Influenza virus pneumonia
Chagas disease (Trypanosoma cruzi)
Leprosy (tuberculoid form)
Cystic fibrosis pneumonitis
Neisserial or pneumococcal meningitis
Filariasis
Post -streptococcal glomerulonephritis
Helicobacter pylori gastritis
Sepsis syndrome
Hepatitis C
Tuberculosis

Diseases of diverse origin in which post-inflammatory fibrosis is a principal cause of pathology:
Bleomycin-induced pulmonary fibrosis
Chronic allograft rejection
Idiopatic pulmonary fibrosis
Hepatic cirrhosis (post -viral or alcoholic)
Radiation-induced pulmonary fibrosis
Schistosomiasis

### Medical conditions

In some embodiments the administering of the compositions of the invention is by intravenous, intramuscular, intraarticular, transcutaneous, subcutaneous, intranasal, peroral, perineural, intrathecal administration, or by local injection or instillation, for example during a surgical procedure.

In some embodiments the administering of the compositions of the invention is by intramuscular administration.

In some embodiments the body fluid preparation is autologous to the mammal in need of said treatment.

In some embodiments the blood serum preparation is autologous to the mammal in need of said treatment.

In some embodiments the blood serum is activated in a vessel comprising an inductor.

In some embodiments the blood serum is activated in a vessel, which does not comprise an inductor.

In some embodiments the inductor is coated on a structure selected from the group consisting of: spheres, gels, glass wool, granulated material and particles or surface structures comprising polystyrene or glass.

In some embodiments the inductor comprises immunoglobulin.

In some embodiments the vessel forms part of a syringe.

In some embodiments the syringe comprises an injection structure suitable for intramuscular injection of the serum directly from the vessel.

In some embodiments the vessel is incubated between 1 and 96 hours, such as between 1 and 22 hours, or between 12 and 96 hours, such as between 12 and 72 hours prior to the administering of the composition contained therein.

In some embodiments the mammal is a human.

In some embodiments the body fluid composition is an activated blood serum preparation.

In some embodiments the body fluid used according to the invention is activated in a process that further comprises a step of treating said body fluid with an anticoagulant.

In some embodiments the method used for the preparation of an activated body fluid composition further comprises a step of separating the blood serum from the blood.

In some embodiments of the invention, the body fluid is an activated blood serum preparation that has been mixed with platelet-rich plasma (PRP).

In some embodiments, the method for preparation of an activated body fluid composition comprises a further step of mixing the activated body fluid composition with platelet-rich plasma (PRP).

In some embodiments the miRNA used according to the invention is selected from the group consisting of hsa-miR-320a, hsa-miR-130a, hsa-miR-320c, hsa-miR-628-3p, hsa-miR-637, hsa-miR-320b, hsa-miR-129-5p, hsa-miR-943, hsa-miR-185*, hsa-miR-340*, hsa-miR-744, hsa-miR-638, hsa-miR-585, hsa-miR-26b, hsa-miR-485-3p, hsa-miR-103, hsa-miR-146b-5p, hsa-miR-642, hsa-miR-146a, hsa-let-7a, hsa-let-7f, hsa-miR-200b*, hsa-miR-320d, hsa-let-7d, hsa-miR-1282, hsa-miR-124, hsa-miR-602, hsa-let-7g, hsa-miR-221, hsa-miR-25*, hsa-miR-1184, hsa-miR-663, hsa-miR-93, hsa-miR-30b*, hsa-miR-124*, hsa-miR-22, hsa-miR-1281, hsa-miR-1237, hsa-miR-34b, hsa-miR-1290, hsa-miR-193b*, hsa-miR-526b, hsa-miR-622, hsa-miR-191, hsa-miR-142-3p, hsa-miR-92a, hsa-miR-1280, hsa-miR-1236, hsa-miR-30c, hsa-miR-877*, hsa-miR-548n, hsa-miR-1249, hsa-let-7i, hsa-miR-1224-3p, hsa-miR-17, hsa-miR-300, hsa-miR-193a-5p, hsa-let-7d*, hsa-miR-24, hsa-miR-518c*, hsa-miR-222, hsa-miR-664, hsa-miR-130b, hsa-miR-625*, hsa-miR-593, hsa-miR-885-5p, hsa-miR-505*, hsa-miR-491-3p, hsa-miR-421, hsa-miR-7, hsa-miR-106a, hsa-miR-99b*, hsa-miR-1300, hsa-miR-92b, hsa-miR-30d, hsa-miR-720, hsa-miR-1260, hsa-miR-425, hsa-miR-939, hsa-miR-30a, hsa-miR-30e, hsa-miR-654-5p, hsa-miR-509-5p, hsa-miR-1826 and variants thereof.

In some embodiments the composition according to the present invention is not derived from a blood product. In some embodiments the composition is essentially free of other blood derived components.

In some embodiments the miRNA is upregulated in a blood preparation upon activation.

In some embodiments the miRNA is upregulated in a body fluid or element thereof upon activation in a vessel comprising an inductor.

In some embodiments the miRNA is upregulated upon activation on a surface selected from the group consisting of: spheres, gels, glass wool, granulated material and particles or surface structures comprising polystyrene or glass.

In some embodiments an inductor used according to the present invention is coated on a structure selected from the group consisting of: spheres, gels, glass wool, granulated material and particles or surface structures comprising polystyrene or glass. In some embodiments the inductor comprises immunoglobulin.

In some embodiments the nucleic acid molecule used in the compositions of the invention is prepared by synthetic means.

In some embodiments the nucleic acid molecule is present in a measurable amount.

In some embodiments the one or more nucleic acid molecule encoding a miRNA present in the composition according to the invention has been upregulated to a 0.00001 to 1000 folds increase, such as a 2 to 1000 folds increase, such as a 10 to 100 folds increase in the measurable amount during activation.

In some embodiments the nucleic acid molecule comprises affinity-enhancing nucleotide analogous, such as a peptide nucleic acid (PNA), pseudo-complementary PNA (pcPNA), locked nucleic acid (LNA) or analogue thereof.

In some embodiments the composition according to the present invention comprises at least 1-100, such as at least 2-50, such as at least 10-50 different nucleic acid molecules encoding each different species of a miRNA.

In some embodiments the composition further comprises a preparation of exosomes. In some embodiments the preparation of exosomes is from the same patient as from where the body fluid or elements thereof derives. In some embodiments the preparation of exosomes is produced by in vitro, such as derived from cells cultured in vitro.

It is to be understood that the miRNA may be incorporated into or on the surface of the exosomes and may protect the miRNA from degradation.

In some embodiments the exosomes are enriched with the nucleic acid molecule according to the present invention.

In some embodiments the body fluid or element thereof according to the present invention is an in vitro cell culture, such as a monocyte cell culture.

In some embodiments the body fluid or element thereof according to the present invention is a blood serum preparation.

In some embodiments the body fluid or element thereof according to the present invention is a buffy coat preparation. In some embodiments the buffy coat preparation further comprises a plasma fraction. In some embodiments the buffy coat preparation with or without the plasma fraction is incubated together with a growth medium prior to or during the activation according to the present invention.

In some embodiments the body fluid or element thereof according to the present invention is whole blood preparation.

In some embodiments the body fluid or element thereof according to the present invention is from bone marrow exudates.

In some embodiments the activated body fluid or element thereof is further mixed with platelet-rich plasma (PRP).

In some embodiments the body fluid or element thereof according to the present invention is activated in a process that further comprises a step of treating said body fluid or element thereof with an anticoagulant, optionally followed by a step of separation, wherein the desired part of the body fluid is isolated.

In some embodiments the body fluid or element thereof according to the present invention is collected from two or more mammals, such as from more than 3, 4, 5, 6, 7, 8, 9, 10, 20, 30, 40, 50, or 100 mammals.

In some embodiments the mammal from where the body fluid is derived is a human.

In some embodiments the mammal is a domestic animal.

In some embodiments the body fluid or element thereof according to the present invention is collected from healthy individual(s).

In some embodiments the body fluid or element thereof according to the present invention is collected from disease individual(s).

In some embodiments the body fluid or element thereof according to the present invention is collected from a combination of healthy and disease individual(s).

In some embodiments the one or more miRNA is upregulated to a concentration level by at least about 50%, such as at least about 100%, such as at least about 200%, such as at least about 300%, such as at least about 400%, such as at least about 500%, such as at least about 600%, such as at least about 700%, such as at least about 800%, as compared to the concentration level of said miRNA in a composition that has not been activated under step b).

In some embodiments the method further comprises a step of incubating the collected body fluid or element thereof in contact with an increased surface area in the presence of synthetic or alternative source of miRNA.

In some embodiments the composition comprising a therapeutically effective amount of one or more nucleic acid molecule encoding a miRNA or functional variant thereof, said miRNA being upregulated in a body fluid or element thereof upon activation of said body fluid or element thereof is a composition as defined as defined herein, or prepared by a method according to the present invention.

In some embodiments the administering is by intravenous, intramuscular, intraarticular, transcutaneous, subcutaneous, intranasal, peroral, perineural, intrathecal administration, or by local injection or instillation, for example during a surgical procedure.

In some embodiments the composition according to the present invention is autologous to the subject in need of said treatment.

In some embodiments the composition according to the present invention is homologous to the subject in need of said treatment.

In some embodiments the composition according to the present invention is heterologous to the subject in need of said treatment.

In some embodiments the composition is prepared by a method according to the present invention, wherein said body fluid or element thereof is incubated between 1 and 100 hours prior to the administering of the composition contained therein.

In some embodiments the subject in need of a treatment according to the present invention is a human.

In some embodiments the medical condition that may be treated by the methods of the present invention is colitis ulcerosa.

In some embodiments the nucleic acid molecule used according to the present invention is a miRNA.

In some embodiments the nucleic acid molecule used according to the present invention is a pri-miRNA.

In some embodiments the nucleic acid molecule used according to the present invention is a pre-miRNA.

In some embodiments the nucleic acid molecule used according to the present invention is a Small interfering RNA (siRNA).

In some embodiments the methods for the treatment or for alleviating the symptoms of a disease or disorder associated with inflammation, a disease of the immune system, such as undesirable activation of the immune system and/or cancer or other indications associated with abnormal cell growth or cell division further comprises the administration of a chemotherapeutic agent.

In some embodiments the chemotherapeutic agent is selected from a group consisting of: alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotecan); bryostatin; callystatin; CC- 1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, caminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6- mercaptopurine, thiamiprine, thioguanine; pyrimidine analogs such as ancitabine, azacitidine, 6- azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., paclitaxel and doxetaxel; chlorambucil; gemcitabine; 6-thioguanine; mercaptopurine; methotrexate; platinum coordination complexes such as cisplatin, oxalip latin and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-I l); topoisomerase inhibitor RFS 2000; difluoromethylomithine (DMFO); retinoids such as retinoic acid; capecitabine; and pharmaceutically acceptable salts, acids or derivatives of any of the above. In another embodiment, the composition of the invention may comprise other biologically active substances, including therapeutic drugs or pro-drugs, for example, other chemotherapeutic agents, scavenger compounds, antibiotics, anti-virals, anti-fungals, antiinflammatories, vasoconstrictors and anticoagulants, antigens useful for cancer vaccine applications or corresponding pro- drugs.

### EXAMPLES

### EXAMPLE 1

### Preparation of activated serum

Blood is collected from either an animal or human being, without any infection or fewer. The blood is taken by a venous transcutane puncture and collected in a container immediately containing the activating substances such as glass beats etc.

After regurgitating or in any other procedure exposing the blood cells to the activating surfaces for 1, minutes, 3, minutes or 4 hours in 37 degrees celcius the sample is left untouched for a total of 12-96 or 12-72 hours depending on the volumen of the blood, the surface area of the vessel and number og leucocytes in the given sample.

Herafter the incubation is terminated by lowering the temperature to room temperature and separating the serum from the clotted blood by filtration or centrifugation. In another aspect of the invention the whole blood is incubated using anticoagulants and after the incubation time the serum is collected by centrifugation or filtration.

The prepared serum or plasma is then stored either at room temperature, frozen at -5-18 degrees celcius or otherwise prepared for freezing storage to optimized the preservation of more complex structures as membrane like vesicles etc.

### EXAMPLE 2

### Alternative procedure for the preparation of activated serum.

Blood is incubated in 60 ml syringes (Perfusor Syringes, Becton Dickinson, USA) over a time period of 6-72 hrs. This process may be enhanced by addition of an inducer. The syringes contain glass beads. Glass beads are 2.5 mm in diameter, have a surface area of 21 mm² and are of medical grade. The beads are washed with sterile, double distilled water until the conductivity is less than 0.3 µS (Hanna Instruments, USA). The surface of the beads is modified by incubation in 50% v/v CrSO4 (Merck, Germany) for 5 min. The beads are then washed repeatedly until the pH is identical to that of the distilled water used for the rinsing and the conductivity is less than 0.3 µS (Hanna Instruments, USA). The syringes are packed with beads and sterilized either by autoclaving or gamma- irradiation.

Blood culture techniques: In all experiments, containers (e.g. 60 ml syringes) packed with beads are filled with freshly drawn human whole blood from healthy, male or female donors, between 20 and 50 years old, without anti-coagulants unless mentioned otherwise. Whole blood cultures are established under sterile, laminar flow conditions (Kendro, Germany). Incubation is carried out aseptically at 37°C, 5% CO₂ (Kendro, Germany) for 24 h intervals. After incubation, serum is retrieved and centrifuged (3500 rpm, 10 min., Megafuge, Kendro, Germany). Alternatively after incubation the serum is separated from the blood cells by centrifugation at 5000 g for 10 min. From the syringes 10 ml serum is retrieved, which corresponds to approximately 20% of the total original blood volume. The serum is stored at -20 °C.

### EXAMPLE 3

### TREATMENT OF HUMAN SUBJECTS WITH ACTIVATED BLOOD SERUM

Patients are given epidural perineural or intramuscular injections three times once a week. Objective and subjective assessments are made at six times (tl-t6) per patient including visual analog scale (VAS) (Joyce et al., Eur J Clin Pharmacol. 14:415-20 (1975)), Oswestry Pain Questionnaire (Fairbank et al. Spine, 25:2940- 2953(2000)), SF-36 (short form health survey) (Ware et al., Med. Care, 30:473-483 (1992)), and standardized clinical examination. 2 ml of activated serum is injected. One group is given activated serum, another is given 10 mg Triamcinolone, and the last is given 5mg Triamcinolone. Triamcinolone is a steroid commonly used to treat inflammation, allergies, arthritis, and asthma. It has been shown that Triamcinolone is effective at reducing lumbar radicular pain, (randomized double blind study, Kramer Eur Spine 1997).

### EXAMPLE 4

5 severe endometriosis patients also having adenomyosis were treated in 6 weeks with conditioned serum prepared as described in example 1. 5 ml of activated serum were administrated to the patient each intramuscular every week for 5-6 weeks. Their symptoms were significantly relieved.

The background for treating intramuscularly was based on several experimental intramuscular injections in 4 horses during the period of a 2 year. This animal study proved that muscular tenderness was cured by the same intervals of injections.

### EXAMPLE 5

2 abortus habitualis patients were treated during In Vitro Fertilisation (IVF) with the same regime as described under example 4, and one conceived during this study.

### EXAMPLE 6

3 colitis ulcerosa and 1 Crohn patients has been through the same timed preparation and injection of activated serum with significant improvement of their conditions/disease, thus in the acute initial treatment a shorter interval for administration is beneficial and after a plateau phase in the symptoms has been reached, a longer interval between administration of the serum seems to stabilize all symptoms. The benefit of this method of sequential strategy for administration of the substances is to enable the invention to cope with the high activity of the disease in the acute phase and changing the activity of the substances when the disease is in a stable phase.

Further to this, the administration of the activated serum has an impact of the course of the disease by administration of a larger volume of conditioned serum in the start of the treatment regimens, such as 5 ml, 10ml or 20 ml.

One patient diagnosed with severe colitis ulcerose having defecation 20 times daily, strictures in the bowel, visualized by coloscopy prior to the treatment with activated serum. The patient was completely cured within 7 weeks (certified by an external doctor performing the coloscopy).

### EXAMPLE 7

In vitro culture of stem cell producing mirRNA including cell cultures of placental origin:
Hematopoietic stem cells or cell cultures of placental origin are cultured under in vitro conditions for a period of time optionally in a container containing the activating substances such as glass beats etc

Alternatively the cell and cell culture medium may in a subsequent step be transferred to a container containing the activating substance such as glass beats etc.

After regurgitating or in any other procedure exposing the cells to the activating surfaces for 1, 2, 4, 10, 24, or 48 hours in 37 degrees Celsius the sample may be left untouched for a total of 5 hour, to 24 hours depending on the volume, the surface area of the vessel and number of cells in a given sample.

Hereafter the incubation is terminated by lowering the temperature to room temperature.

The prepared cell preparation may then stored either at room temperature, frozen at -5-18 degrees Celsius or otherwise prepared for freezing storage to optimized the preservation of more complex structures as membrane like vesicles etc.

### EXAMPLE 8

Activated serum as prepared by examples 1 or 2 was tested in an assay to determine the specific miRNAs upregulated in response to the activation. A miRCURY™ LNA Array microRNA Profiling Service was performed by Exiqon (Denmark). Results are summarized in table 2.

Table 2 contains normalised Hy3 signals (log2 transformed) from all hybridizations. Shown is the median of replicated measurements of the same miRNA from each slide.

**Table 2**

| | Log2-transformed values | | Normalized signal medianvalues | | | |
|---|---|---|---|---|---|---|
| **Call rate** | 7% | 7% | | | Delta Log2-transformed values | Fold change |
| | Serum | Activated serum (1/5) | Serum | Activated serum (1/5) | | |
| | | | | | | |

| **Annotation** | Slide 1 | Slide 2 | Slide 1 | Slide 2 | Activated serum (1/5) versus vs Serum | Activated serum (1/5) versus vs Serum |
|---|---|---|---|---|---|---|
| hsa-miR-320a | 6,93 | 8,20 | 122 | 293 | 1,26 | 2,40 |
| hsa-miR-130a | 5,83 | 7,08 | 57 | 135 | 1,24 | 2,37 |
| hsa-miR-320c | 6,98 | 8,20 | 126 | 295 | 1,23 | 2,34 |
| hsa-miR-628-3p | 7,07 | 8,15 | 134 | 284 | 1,08 | 2,11 |
| hsa-miR-637 | 7,81 | 8,88 | 224 | 473 | 1,08 | 2,11 |
| hsa-miR-320b | 7,27 | 8,31 | 154 | 318 | 1,04 | 2,06 |
| hsa-miR-129-5p | 9,86 | 10,90 | 929 | 1912 | 1,04 | 2,06 |
| hsa-miR-943 | 8,20 | 9,20 | 295 | 586 | 0,99 | 1,99 |
| hsa-miR-185* | 7,88 | 8,87 | 236 | 467 | 0,99 | 1,98 |
| hsa-miR-340* | 6,11 | 7,06 | 69 | 133 | 0,95 | 1,93 |
| hsa-miR-744 | 7,05 | 7,98 | 132 | 252 | 0,93 | 1,90 |
| hsa-miR-638 | 9,89 | 10,81 | 946 | 1796 | 0,93 | 1,90 |
| hsa-miR-585 | 8,03 | 8,95 | 261 | 494 | 0,92 | 1,89 |
| hsa-miR-26b | 6,69 | 7,54 | 103 | 186 | 0,85 | 1,81 |
| hsa-miR-485-3p | 8,20 | 9,01 | 294 | 516 | 0,81 | 1,75 |
| hsa-miR-103 | 5,49 | 6,28 | 45 | 78 | 0,78 | 1,72 |
| hsa-miR-146b-5p | 5,70 | 6,44 | 52 | 87 | 0,74 | 1,67 |
| hsa-miR-642 | 6,28 | 7,02 | 78 | 130 | 0,74 | 1,66 |
| hsa-miR-146a | 5,50 | 6,23 | 45 | 75 | 0,73 | 1,66 |
| hsa-let-7a | 5,87 | 6,60 | 58 | 97 | 0,73 | 1,66 |
| hsa-let-7f | 6,69 | 7,42 | 103 | 171 | 0,73 | 1,66 |
| hsa-miR-200b* | 7,54 | 8,27 | 186 | 308 | 0,73 | 1,65 |
| hsa-miR-320d | 7,22 | 7,93 | 149 | 243 | 0,70 | 1,63 |
| hsa-let-7d | 5,64 | 6,23 | 50 | 75 | 0,59 | 1,50 |
| hsa-miR-1282 | 5,53 | 6,12 | 46 | 69 | 0,58 | 1,50 |
| hsa-miR-124 | 6,54 | 7,11 | 93 | 138 | 0,57 | 1,49 |
| hsa-miR-602 | 10,00 | 10,54 | 1023 | 1489 | 0,54 | 1,46 |
| hsa-let-7g | 5,70 | 6,23 | 52 | 75 | 0,53 | 1,44 |
| hsa-miR-221 | 6,01 | 6,54 | 64 | 93 | 0,53 | 1,44 |
| hsa-miR-25* | 9,02 | 9,54 | 521 | 744 | 0,52 | 1,43 |
| hsa-miR-1184 | 6,65 | 7,16 | 100 | 143 | 0,51 | 1,43 |
| hsa-miR-663 | 6,00 | 6,48 | 64 | 89 | 0,48 | 1,39 |
| hsa-miR-93 | 6,30 | 6,78 | 79 | 110 | 0,47 | 1,39 |
| hsa-miR-30b* | 5,95 | 6,39 | 62 | 84 | 0,44 | 1,36 |
| hsa-miR-124* | 6,66 | 7,10 | 101 | 138 | 0,44 | 1,36 |
| hsa-miR-22 | 8,80 | 9,23 | 445 | 601 | 0,43 | 1,35 |
| hsa-miR-1281 | 5,63 | 6,06 | 50 | 67 | 0,42 | 1,34 |
| hsa-miR-1237 | 5,66 | 6,08 | 51 | 68 | 0,42 | 1,34 |
| hsa-miR-34b | 6,40 | 6,82 | 84 | 113 | 0,42 | 1,34 |
| hsa-miR-1290 | 12,98 | 13,39 | 8064 | 10719 | 0,41 | 1,33 |
| hsa-miR-193b* | 8,04 | 8,43 | 264 | 344 | 0,38 | 1,30 |
| hsa-miR-526b | 5,53 | 5,91 | 46 | 60 | 0,38 | 1,30 |
| hsa-miR-622 | 5,79 | 6,17 | 55 | 72 | 0,37 | 1,30 |
| hsa-miR-191 | 7,74 | 8,11 | 214 | 276 | 0,36 | 1,29 |
| hsa-miR-142-3p | 5,63 | 5,98 | 49 | 63 | 0,35 | 1,27 |
| hsa-miR-92a | 5,89 | 6,23 | 59 | 75 | 0,34 | 1,26 |
| hsa-miR-1280 | 7,68 | 8,02 | 206 | 260 | 0,34 | 1,26 |
| hsa-miR-1236 | 5,85 | 6,18 | 58 | 72 | 0,33 | 1,26 |
| hsa-miR-30c | 5,85 | 6,17 | 58 | 72 | 0,32 | 1,24 |
| hsa-miR-877* | 5,71 | 6,02 | 52 | 65 | 0,31 | 1,24 |
| hsa-miR-548n | 5,54 | 5,85 | 47 | 58 | 0,31 | 1,24 |
| hsa-miR-1249 | 6,24 | 6,54 | 75 | 93 | 0,30 | 1,23 |
| hsa-let-7i | 5,90 | 6,19 | 60 | 73 | 0,30 | 1,23 |
| hsa-miR-1224-3p | 5,72 | 6,01 | 53 | 64 | 0,28 | 1,22 |
| hsa-miR-17 | 5,83 | 6,11 | 57 | 69 | 0,28 | 1,21 |
| hsa-miR-300 | 6,14 | 6,42 | 70 | 85 | 0,28 | 1,21 |
| hsa-miR-193a-5p | 5,54 | 5,81 | 47 | 56 | 0,27 | 1,21 |
| hsa-let-7d* | 5,52 | 5,78 | 46 | 55 | 0,27 | 1,20 |
| hsa-miR-24 | 7,43 | 7,69 | 172 | 207 | 0,26 | 1,20 |
| hsa-miR-518c* | 5,62 | 5,88 | 49 | 59 | 0,26 | 1,20 |
| hsa-miR-222 | 5,52 | 5,76 | 46 | 54 | 0,24 | 1,18 |
| hsa-miR-664 | 5,94 | 6,18 | 62 | 73 | 0,24 | 1,18 |
| hsa-miR-130b | 6,26 | 6,48 | 76 | 89 | 0,22 | 1,17 |
| hsa-miR-625* | 5,97 | 6,19 | 63 | 73 | 0,22 | 1,16 |
| hsa-miR-593 | 5,46 | 5,68 | 44 | 51 | 0,22 | 1,16 |
| hsa-miR-885-5p | 5,55 | 5,74 | 47 | 53 | 0,18 | 1,14 |
| hsa-miR-505* | 5,73 | 5,90 | 53 | 60 | 0,17 | 1,13 |
| hsa-miR-491-3p | 5,67 | 5,84 | 51 | 57 | 0,17 | 1,12 |
| hsa-miR-421 | 5,70 | 5,87 | 52 | 58 | 0,16 | 1,12 |
| hsa-miR-7 | 6,33 | 6,49 | 81 | 90 | 0,16 | 1,12 |
| hsa-miR-106a | 5,59 | 5,75 | 48 | 54 | 0,16 | 1,12 |
| hsa-miR-99b* | 6,57 | 6,73 | 95 | 106 | 0,16 | 1,11 |
| hsa-miR-1300 | 6,01 | 6,15 | 65 | 71 | 0,13 | 1,10 |
| hsa-miR-92b | 5,88 | 6,01 | 59 | 65 | 0,13 | 1,09 |
| hsa-miR-30d | 5,89 | 6,02 | 59 | 65 | 0,13 | 1,09 |
| hsa-miR-720 | 10,99 | 11,11 | 2037 | 2212 | 0,12 | 1,09 |
| hsa-miR-1260 | 5,50 | 5,61 | 45 | 49 | 0,11 | 1,08 |
| hsa-miR-425 | 5,63 | 5,69 | 50 | 52 | 0,06 | 1,04 |
| hsa-miR-939 | 8,51 | 8,55 | 363 | 374 | 0,04 | 1,03 |
| hsa-miR-30a | 6,78 | 6,82 | 110 | 113 | 0,03 | 1,02 |
| hsa-miR-30e | 5,96 | 5,99 | 62 | 64 | 0,03 | 1,02 |
| hsa-miR-654-5p | 5,76 | 5,78 | 54 | 55 | 0,02 | 1,01 |
| hsa-miR-509-5p | 6,49 | 6,51 | 90 | 91 | 0,02 | 1,01 |
| hsa-miR-1826 | 10,16 | 10,18 | 1148 | 1157 | 0,01 | 1,01 |

### EXAMPLE 9

Activated serum as prepared by examples 1 or 2, wherein the samples were incubated for a period of at least 36 hours, such as 72 hours, was tested by quantitative PCR in an assay to determine the specific miRNAs upregulated in response to the activation. Results, showing the up-regulation as compared to a control that has not been activated, are summarized for a small non-limiting selection of miRNAs in table 3.

**Table 3**

| MiRNA: | Up-regulation (times the amount of control) |
|---|---|
| hsa-let-7a | 28 |
| hsa-let-7b | 25 |
| hsa-let-7b* | 3.6 |
| hsa-let-7c | 24 |
| hsa-let-7d | 28 |
| hsa-let-7e | 2 |
| hsa-let-7f | 28 |
| hsa-let-7g | 12 |
| hsa-let-7i | 17 |
| hsa-miR-18a | 14 |
| hsa-miR-205 | 6,7 |
| hsa-miR-126 | 1.7 |
| hsa-miR-34a | 4 |
| hsa-miR-29c | 2 |
| hsa-miR-133a | 6 |
| hsa-miR-15a | 21 |
| hsa-miR-15b | 12.8 |
| hsa-miR-15b* | 11.8 |
| hsa-miR-16 | 14.8 |
| | |
| | |
| | |

## Claims

1. An activated blood serum preparation or an activated whole blood preparation for use in the treatment or for alleviating the symptoms of colitis ulcerosa in a mammal.

2. The activated blood serum preparation or the activated whole blood preparation for use according to claim 1, wherein the blood serum preparation is autologous, allogenic or xenogenic to the mammal in need of said treatment.

3. The activated blood serum preparation or the activated whole blood preparation for use according to any of the preceding claims, wherein the blood serum is activated in a vessel.

4. The activated blood serum preparation or the activated whole blood preparation for use according to claim 3, wherein said vessel contain an inductor coated on a structure selected from the group consisting of: spheres, gels, glass wool, granulated material and particles or surface structures comprising polystyrene or glass.

5. The activated blood serum preparation or the activated whole blood preparation for use according to any of claims 3-4, wherein the vessel forms part of a syringe.

6. The activated blood serum preparation or the activated whole blood preparation for use according to any of claims 3-5, wherein the vessel is incubated between 1 and 96, such as between 1 and 76, such as between 12 and 72 hours prior to the administering of the composition contained therein.

7. A composition comprising an activated blood serum preparation or an activated whole blood preparation for use in the treatment or for alleviating the symptoms of colitis ulcerosa in a mammal.

## Patentansprüche

1. Aktiviertes Blutserumpräparat oder aktiviertes Vollblutpräparat für die Verwendung in der Behandlung oder zur Linderung der Symptome von Colitis ulcerosa bei einem Säugetier.

2. Aktiviertes Blutserumpräparat oder aktiviertes Vollblutpräparat für die Verwendung nach Anspruch 1, wobei das Blutserumpräparat autolog, allogen oder xenogen zu dem Säugetier ist, das eine derartige Behandlung benötigt.

3. Aktiviertes Blutserumpräparat oder aktiviertes Vollblutpräparat für die Verwendung nach einem der vorhergehenden Ansprüche, wobei das Blutserum in einem Behältnis aktiviert wird.

4. Aktiviertes Blutserumpräparat oder aktiviertes Vollblutpräparat für die Verwendung nach Anspruch 3, wobei das Behältnis einen Induktor enthält, der auf eine Struktur aufgetragen ist, die ausgewählt ist aus der Gruppe, bestehend aus: Kugeln, Gelen, Glaswolle, granuliertem Material und Partikeln oder Oberflächenstrukturen, die Polystyrol oder Glas umfassen.

5. Aktiviertes Blutserumpräparat oder aktiviertes Vollblutpräparat für die Verwendung nach einem der Ansprüche 3 bis 4, wobei das Behältnis ein Teil einer Spritze bildet.

6. Aktiviertes Blutserumpräparat oder aktiviertes Vollblutpräparat für die Verwendung nach einem der Ansprüche 3 bis 5, wobei das Behältnis zwischen 1 und 96, wie beispielsweise zwischen 1 und 76, wie beispielsweise zwischen 12 und 72 Stunden vor der Verabreichung der darin enthaltenen Zusammensetzung inkubiert wird.

7. Zusammensetzung, die ein aktiviertes Blutserumpräparat oder ein aktiviertes Vollblutpräparat für die Verwendung in der Behandlung oder zur Linderung der Symptome von Colitis ulcerosa bei einem Säugetier umfasst.

## Revendications

1. Préparation de sérum sanguin activé ou une préparation de sang total activé pour son utilisation dans le traitement ou le soulagement des symptômes de la colite ulcéreuse chez un mammifère.

2. Préparation de sérum sanguin activé ou la préparation de sang total activé pour son utilisation selon la revendication 1, dans laquelle la préparation de sérum sanguin est autologue, allogénique ou xénogénique relativement au mammifère nécessitant ledit traitement.

3. Préparation de sérum sanguin activé ou la préparation de sang total activé pour son utilisation selon l'une quelconque des revendications précédentes, dans laquelle le sérum sanguin est activé dans un récipient.

4. Préparation de sérum sanguin activé ou la préparation de sang total activé pour son utilisation selon la revendication 3, dans laquelle ledit récipient contient un inducteur revêtu sur une structure sélectionnée dans le groupe constitué de : sphères, gels, laine de verre, matériau granulé et particules ou structures superficielles comprenant du polystyrène ou du verre.

5. Préparation de sérum sanguin activé ou la préparation de sang total activé pour son utilisation selon l'une quelconque des revendications 3 à 4, dans laquelle le récipient fait partie d'une seringue.

6. Préparation de sérum sanguin activé ou la préparation de sang total activé pour son utilisation selon l'une quelconque des revendications 3 à 5, dans laquelle le récipient est mis à incuber de 1 à 96 heures, par exemple de 1 à 76 heures, par exemple de 12 à 72 heures avant l'administration de la composition y étant contenue.

7. Composition comprenant une préparation de sérum sanguin activé ou une préparation de sang total activé pour son utilisation dans le traitement ou le soulagement des symptômes de la colite ulcéreuse chez un mammifère.
